Europäisches Patentamt

European Patent Office

Office européen des brevets

(19)

(11) Publication number: **0 225 160 B1**

(12) **EUROPEAN PATENT SPECIFICATION**

(45) Date of publication of patent specification: **21.08.91**    (51) Int. Cl.⁵: **A61K 35/16, A61K 37/02**

(21) Application number: **86309197.1**

(22) Date of filing: **25.11.86**

The file contains technical information submitted
after the application was filed and not included in
this specification

(54) **Compositions and methods for the treatment of bleeding disorders.**

(30) Priority: **26.11.85 DK 5446/85**
**26.09.86 DK 4592/86**

(43) Date of publication of application:
**10.06.87 Bulletin  87/24**

(45) Publication of the grant of the patent:
**21.08.91 Bulletin  91/34**

(84) Designated Contracting States:
**AT BE CH DE ES FR GB GR IT LI LU NL SE**

(56) References cited:
**WO-A-81/02105**
**WO-A-83/00016**
**US-A- 4 357 321**
**US-A- 4 470 969**
**US-A- 4 473 553**

(73) Proprietor: **NOVO NORDISK A/S**
**Novo Allé**
**DK-2880 Bagsvaerd(DK)**

(72) Inventor: **Hedner, Ulla Karin Elisabeth**
**Bagängsvägen 29**
**SE-21620 Malmo(SE)**

(74) Representative: **Brown, John David et al**
**FORRESTER & BOEHMERT Widenmayer-**
**strasse 4/I**
**W-8000 München 22(DE)**

Note: Within nine months from the publication of the mention of the grant of the European patent, any person
may give notice to the European Patent Office of opposition to the European patent granted. Notice of opposition
shall be filed in a written reasoned statement. It shall not be deemed to have been filed until the opposition fee
has been paid (Art. 99(1) European patent convention).

Rank Xerox (UK) Business Services

## Description

Technical Field

The present invention is generally directed toward the use of factor VIIa for treatment of patients suffering from bleeding disorders such as platelet disorders including thrombocytopenia, and others typically present in association with severe tissue damages. In accordance with the present invention, factor VIIa may also be used for the treatment of gastrointestinal bleedings and nasal-oral bleedings even in cases where no specific basic haemostatic disorders have been diagnosed.

Background Art

Uncontrolled and excessive bleeding is a major problem both in connection with surgery and also various forms of tissue damages. Bleeding disorders may be caused by clotting factor deficiencies or clotting factor inhibitors (haemophilia A and B). Bleeding disorders are, however, also seen in patients not suffering from haemophilia A or B, for example in patients suffering from von Willebrand's disease. Patients with von Willebrand's disease have a defective primary haemostasis because they lack or have an abnormal von Willebrand factor protein. Bleeding disorders are also seen in patients with a normally functioning blood clotting cascade and may be caused by a defective platelet function, thrombocytopenia, or even by unknown reasons.

Clot formation is basically induced by the conversion of the soluble plasma protein fibrinogen into insoluble fibrin catalyzed by the enzyme thrombin. The blood components which participate in the coagulation cascade are proenzymes or zymogens, enzymatically inactive proteins which are converted to proteolytic enzymes by the action of an activator, itself an activated clotting factor. Coagulation factors which have undergone such a conversion are generally referred to as "activated factors", and are designated by the addition of a lower case postscript "a" (e.g., VIIa).

There are two separate systems which can promote blood clotting. These systems are referred to as the intrinsic and the extrinsic coagulation pathway. In the intrinsic pathway only factors present in plasma are utilized. An intermediate event in the intrinsic pathway is the activation of factor IX to factor IXa, a reaction catalyzed by factor XIa and calcium ions. Factor IXa then participates in the activation of factor X to factor Xa in the presence of factor VIIIa, phospholipid and calcium ions. The extrinsic pathway involves plasma factors as well as components present in tissue extracts Factor VII, one of the proenzymes referred to above, participates in the extrinsic pathway of blood coagulation by converting (upon its activation to VIIa) factor X to Xa in the presence of tissue factor and calcium ions. Factor Xa in turn then converts prothrombin to thrombin in the presence of factor Va, calcium ions and phospholipid Because the activation of factor X to factor Xa is an event shared by both the intrinsic and extrinsic pathways, factor VIIa may be used for the treatment of patients with deficiencies or inhibitors of factor VIII (U.S. Patent No. 4,382,083) and factor VIIa has been shown to be capable of by-passing the initial phase of the coagulation casade in haemophilia A patients with antibodies against VIII:C, Hedner and Kisiel, J.Clin.Invest., 71:1836 - 1841,1983.

Thrombocytopenia, defined as "a decreased number of circulating platelets," is a common clinical problem associated with a diverse group of diseases and complex situations in which multiple factors contribute to the low platelet count. Lowered platelet counts will result in an increased bleeding tendency manifesting itself in mucosal bleedings from, for example the nasal-oral area or the gastrointestinal tract, as well in oozing from wounds, ulcers and injection sites. Thrombocytopenic bleedings can be extensive and create serious problems both during surgery and also postoperatively. Even minor surgery such as tooth extractions may cause severe bleedings. Furthermore, spontaneous intracranial bleedings may occur at extremely low platelet counts ($<10 \times 10^9$/l).

A decreased number of circulating platelets may be the result of: (l) a production defect, (2) an abnormal distribution, (3) a dilutional loss (massive blood transfusion), or (4) an abnormal destruction.

A defective production of platelets in the bone marrow may be the result of a variety of conditions including the influence of toxic agents such as irradiation, cytostatics, certain drugs, etc., tumour infiltrations (metastatic cancer and leukaemia) or degenerative processes of unknown origin (often associated with anaemia or other blood disorders). An abnormal distribution of platelets is seen in association with haemotologic disorders (leukaemia, myeloma, lymphoma), liver diseases, tumours, etc. In these situations the platelets may be trapped in an enlarged spleen or liver and thus escape from the circulating blood. Massive blood transfusion without special addition of fresh platelets will result in a lowered concentration of platelets in the circulating blood and is thought to be the cause of thrombocytopenic bleedings that may occur in such situations. An abnormal destruction of platelets may be the result of: (l) an increased consumption in vessel grafts or in traumatized tissue or (2) an immune mechanism as may be seen in drug-induced thrombocytopenia, idiopathic thrombocytopenic purpura (ITP), autoimmune disease, haematologic disorders (leukaemia, lymphoma), etc.

Platelets are of importance for the primary haemostasis by inducing the formation of a primary haemostatic plug that subsequently is solidified through the activation of the coagulation cascade and the formation of fibrin. The platelets normally provide coagulation factors including factor V, factor VII, and fibrinogen as well as phospholipids that are necessary for the initiation of local haemostasis.

In patients suffering from thrombocytopenia the normal coagulation cascade is put out of function due to lack of the initiation of the primary steps of the coagulation cascade. The treatment of such patients meet with substantial difficulties. Patients with thrombocytopenia are at present most commonly treated by the administration of platelet concentrates prepared from donor blood. Such concentrations consist of pooled platelets from 5 - 6 donors. Most repeated recipients of platelets transfusions develop antibodies against platelet antigens, resulting in a poor or totally absent effect of further platelet transfusions. There is presently no treatment to be offered such patients.

A defective platelet function is rather common both as a congenital disorder (Glanzmann's thrombastenia, other congenital forms of thrombastenia, platelet aggregation defects) and as a complication to a number of diseases such as leukaemia, dysproteinaemia (e.g. myeloma), autoimmune diseases (rheumatoid arthritis, systemic lupus erythematosus, etc.) and uraemia. Patients with a defective platelet function may develop bleedings mostly of mucosal type as described above for those with thrombocytopenia. In association with surgery these patients also need treatment to avoid excessive bleeding. Currently antifibrinolytic treatment (tranexamic acid, $\epsilon$ -aminocaproic acid) is used alone or together with the administration of desmopressin (DDVAP), a vasopressin analogue. However, desmopressin also has cardiovascular effects resulting in vasoconstriction. This makes the drug unsuitable for use in patients suspected to have some sort or cardiovascular problems.

WO-A-8300016 discloses treatment of bleeding disorders caused by clotting factor defects and inhibitors.

Consequently, there is a need in the art for an improved method of treating patients with a defective platelet function, as well as those patients suffering from thrombocytopenia, which method does not suffer from the unwanted side effects and inconveniences characteristic or prior treatments. The present invention fulfills this need and further provides other related advantages, including a method for treating gastrointestinal and nasal-oral bleedings, even in situations where no specific haemostatic disorders have been diagnosed.

Disclosure of the Invention

According to the present invention there is provided a use of factor VIIa in the preparation of a composition for treating patients suffering from bleeding disorders not caused by clotting factor defects or clotting factor inhibitors, which composition comprises factor VIIa in an effective haemostatic amount.

Briefly stated, there is disclosed a method for treating patients suffering from bleeding disorders not caused by clotting factor defects or clotting factor inhibitors. The method generally comprises administering to the patient a composition comprising an effective haemostatic amount of factor VIIa. The composition may also include a physiologically acceptable carrier or diluent, or and adjuvant. Suitable adjuvants include albumin, calcium, non-reducing sugars, polyalcohols, polysaccharides and antioxidants.

The method set forth herein is particularly effective in treating patients suffering from thrombocytopenia, as well as other platelet disorders. In addition, the method may be used to treat patients suffering from gastrointestinal bleedings or nasal-oral bleedings.

In a preferred embodiment of the method of the present invention, the composition is administered intravenously, and in an amount from about 100 units to 1000 units, more preferably 100 to 500 units of factor VIIa per 5 kilogram of body weight. The composition is preferably administered during a time period of approximately 24 hours.

A composition suitable for use in treating bleeding disorders as well as the methods described herein preferably comprises purified factor VIIa in a concentration of at least 25 $\mu$g/ml.

It will be appreciated by those skilled in the art that, for ease of administration, it is preferable to utilize a concentration of factor VIIa of approximately 25 $\mu$g/ml - 500 $\mu$g/ml, and more preferably, a concentration of 25 $\mu$g/ml - 200 $\mu$g/ml, although significantly higher concentrations could be used within the present invention. The use of concentrations as described above allows convenient infusions of from 1 - 5 ml per dose.

Other aspects of the present invention will become evident upon reference to the following detailed description.

## Best Mode for Carrying out the Invention

In its broadest aspect the present invention provides a method for treating patients suffering from bleeding disorders not caused by clotting factor deficiencies or clotting factor inhibitors. Within this method, a composition which includes an activated haemostatic agent containing an effective amount of factor VIIa is administered to the patient.

The composition may contain unactivated factor VII and other unactivated blood coagulation factors, such as factor IX, which may enhance the activity of factor VIIa. The factor IX concentration should preferably be in a range that corresponds to a given dose of about 10 units per kilogram body weight. It is preferred that factor VIIa be unaccompanied by blood coagulation factors other than factor IX.

Human purified factor VIIa is preferably made by the methods described by Broze and Majerus, J.Biol.Chem. 255 (4): 1242 - 1247, 1980 and Hedner and Kisiel, J.Clin.Invest. 71: 1836 - 1841, 1983. These methods yields factor VII without detectable amounts of other blood coagulation factors. An even further purified factor VII preparation may be obtained by including an additional gel filtration as the final purification step. Factor VII is then converted into activated factor VIIa by known means, e.g. by several different plasma proteins, such as factor XIIa, IXa or Xa. Alternatively, as described by Bjoern et al. (Research Disclosure, 269 September 1986, pp. 564 - 565), factor VII may be activated by passing it through an ion-exchange chromatography column, such as Mono Q® (Pharmacia Fine Chemicals) or the like. It will be appreciated by those skilled in the art that a suitable factor VIIa for use in the present invention may also be produced by DNA recombinant technology, e.g. by insertion of the cDNA or gene encoding factor VII (Hagen et al., Proc.Natl.Acad.Sci. USA 83: 2412 - 2416, 1986) in a suitable vector, transforming of suitable cell lines with the vector and growing the transformed cells in an appropriate medium whereupon the expressed product is isolated and activated into factor VIIa. Factor VIIa produced by DNA recombinant technology may be authentic factor VIIa or a more or less modified factor VIIa provided that such modified factor VIIa has substantially the same biological activity for blood coagulation as autenthic factor VIIa. Such modified factor VIIa may be prepared by modifying the DNA sequence encoding factor VII either by altering the amino acid codons or by removal of some of the amino acid codons in the natural gene by known means, e.g. by site specific mutagenesis.

It is evident that the practice of the methods described herein is independent of how the purified factor VIIa is derived and, therefore, the present invention is contemplated to cover use of any factor VIIa preparation suitable for use herein.

According to the present invention factor VIIa is shown to be capable of arresting bleeding in patients that have virtually no circulating platelets. Briefly, purified factor VIIa was injected into rabbits made thrombocytopenic by anti-platelet serum, and the experiments demonstrated that trace amounts of purified human factor VIIa effectively arrested bleeding in the thrombocytopenic animals. Factor VIIa is thus capable of by-passing the primary haemostasis and may cause local haemostasis without the participation of platelets and the initial coagulation phase. Factor VIIa is also shown to be able to induce local haemostasis in human patients suffering from thrombocytopenia.

Patients suffering waste tissue damage with a massive cell destruction may develop complex haemostatic disorders as a result of the release of a variety of enzymes from the disrupted cells. Such enzymes may influence both the coagulation and fibrinolytic systems, leading to a degradation of several factors involved with one system or the other. It may also be beneficial to use factor VIIa in these patients due to the capability of VIIa to produce a haemostatic plug through activation of the latter phases of the coagulation system. Treatment using factor VIIa in this regard may be through intravenous injection or application locally, and may be combined with anti-fibrinolytic therapy. Further, in any bleeding situation, e.g., gastrointestinal or nasal-oral bleedings or in surgery, it may be beneficial to use factor VIIa in substantially the same concentrations as described herein, thus inducing local haemostasis. Factor VIIa may be applied locally or intravenously in these situations.

Factor VIIa is preferably administered by intravenous injections and in an amount of about 100 - 1000 units per kg body weight, and preferably in an amount of about 100 - 500 units per kg body weight corresponding to 5 about 2 - 5 $\mu$g/kg, a dose that may have to be repeated 2 - 4 times/24 hours.

"One unit", as used herein, is defined as the amount of factor VII present in 1 ml of normal plasma, corresponding to about 0.5 $\mu$g protein. After activation 50 units corresponds to about 1 $\mu$g protein.

A "haemostatic effect" or "amount," as used herein, is defined as the substantial cessation of bleeding within 15 minutes after administration of about 100 - 1000 units per kg body weight of pure factor VIIa.

There is also disclosed a method for the preparation of a pharmaceutical composition for the treatment of bleeding disorders in which factor VIIa, preferably in purified form, is mixed with suitable adjuvants or a suitable carrier or diluent. Suitable physiologically acceptable carriers or diluents include sterile water and saline. Suitable adjuvants, in this regard, include calcium, albumins or other inert proteins to stabilize the purified factor VII a. Other physiologically acceptable adjuvants are non-reducing sugars, polyalcohols (such as sorbitol or glycerol), polysaccharides such as low molecular weight dextrins, amino acids and antioxidants (such as bisulfite and ascorbate). The adjuvants are generally present in a concentration of from 0.1 to 3% w/v. The pharmaceutical composition may also contain protease inhibitors, e.g. aprotinin. In a particularly preferred embodiment, calcium is used in a combination with another selected adjuvant within the pharmaceutical composition. The amount of calcium is preferably 5 - 50 mM and more preferably 10 - 20 mM.

The following examples are offered by way of illustration, and not by way of limitation.

Example I

Rabbits were made thrombocytopenic by the administration of sheep antibodies against rabbit platelets prepared by the methods of Busch et al., Acta Chir.Scand., I40:255, I974. Haemostatic plug formation in the rabbit mesenteric microvessels was studied by the method of Bergqvist and Arfors, Thromb.Diathes Haemorrh , 30:586, I973. For each observation time three arterioles and three venules (diameter 20 - 40 $\mu$m) were transected and the time for haemostatic plug formation was measured and the frequency of rebleeding recorded. The time required for primary haemostatic plug formation was defined as "the interval between transection and the first arrest of bleeding". The sum of this and all the rebleeding times was called the "total haemostatic plug formation time" (THT). The platelet counts in the rabbits decreased to a minimum I5 - 60 minutes after the antiplatelet serum was administrated and remained low throughout the observation period. In the control animals, a decrease from 263 $\times$ I0$^9$/l (mean value) to I0 $\times$ I0$^9$/l (mean value) occurred. Before the platelet antibodies were administrated, the THT in the arterioles (THT-A) showed a mean of 54 s. A greater than three-fold prolongation of THT-A to I79 seconds was observed I5 minutes as well as 60 minutes after the antibody administration. The THT in the venules (THT-V) varied between 202 and 394 s (mean 274 s) prior to antibody administration. In parallel with the prolongation of THT-A, a prolongation of the THT-V (mean 768 s) I5 minutes after the antibody administration was observed and stayed relatively constant throughout the observation time.

Three rabbits were made thrombocytopenic in the same way as the control animals and human Factor VIIa (50 units per kg body weight) was then administrated (30 minutes after the antibody). Ten minutes after the factor VIIa was injected the THT-A values showed a substantial shortening in each rabbit (mean II4 s; mean after the antibody administration 260 s prior to factor VIIa). The shortening was, however, transient (mean 30 minutes after the factor VIIa was 2I9 s). The THT-V showed a similar pattern with a shortening I0 minutes after the factor VIIa (mean after the antibody administration 698 s and I0 minutes after the factor VIIa 499 s). The slight shortening was transient; and 30 minutes after the factor VIIa injection, the THT-V showed a mean of 672 s.

Another 5 thrombocytopenic rabbits were then given twice as much factor VIIa (I00 units per kg body weight) and ten minutes after the factor VIIa was given, the THT-A showed a marked shortening from a mean of 256 s after the antibody administration to a mean of 89 s ten minutes after the factor VIIa. This shortening persisted throughout the observation time. The THT-V was markedly shortened following the injection of factor VIIa (from a mean of 59I s after the antibody administration to a mean of 390 s). After 30 minutes a normalization of the THT-V had occurred (mean 255 s) and the same was observed after 60 minutes (mean 299 s).

No effect on the THTs was noted in 5 non-thrombocytopenic rabbits. When factor VII was given rather than factor VIIa no effect on the THT was seen.

The results are summarized in the following table.

Table

The THT-A and THT-V in 5 rabbits before and after APS followed by administration of factor VIIa (100 u/kg b.w.). The means of 3 transsections at each check point are given. The numbers within parantheses are the values obtained in 3 thrombocytopenic rabbits given factor VII in a dose of 60 u/kg b.w.

| Rabbit No. | Before | 15 minutes after APS | 30 minutes after APS / 10 minutes after Factor VIIa (VII) (100 u/kg b.w.) | 60 minutes after APS / 30 minutes after Factor VIIa (VII) (100 u/kg b.w.) | 90 minutes after APS / 60 minutes after Factor VIIa (VII) (100 u/kg b.w.) |
|---|---|---|---|---|---|
| THT-A 1 | 42 (77) | 131 (225) | 89 (288) | 111 (144) | – |
| (sec) 2 | 63 (57) | 483 (222) | 104 (256) | 153 (87) | 86 |
| 3 | 90 (75) | 206 (763) | 81 (717) | 119 (631) | 210 |
| 4 | 61 | 179 | 77 | 50 | 72 |
| 5 | 53 | 279 | 95 | 95 | 110 |
| mean | 65 (70) | 256 (403) | 89 (420) | 106 (364) | 102 |
| THT-V 1 | 277 (176) | 719 (580) | 485 (900) | 253 (411) | – |
| 2 | 164 (321) | 463 (768) | 357 (812) | 187 (657) | 236 |
| 3 | 254 (230) | 584 (899) | 274 (900) | 288 (839) | 243 |
| 4 | 213 | 599 | 551 | 155 | 229 |
| 5 | 376 | 590 | 285 | 391 | 487 |
| mean | 257 (242) | 591 (749) | 390 (871) | 255 (636) | 299 |
| Platelet count x 10$^9$/l mean | 222 (193) | 2 (6) | 2 (8) | 6 (25) | 10 |
| range | 243-214 (93-283) | 0-8 (2-9) | 1-2 (2-12) | 5-10 (18-23) | 7-12 |

The present experiments demonstrate the important role of factor VIIa in the initiation of the coagulation process in vivo. In contrast, factor VII had little if any effect in this process. Furthermore, factor VIIa was capable of initiating the coagulation process in the absence of platelets Accordingly, the phospholipid normally provided at the site of injury by the platelets is made available from damaged endothelial cells that also provide the tissue factor.

Example 2

Treatment of 2 patients with thrombocytopenia using pure factor VIIa

Two patients having haematological disorders (macroglobulinaemia Waldenström and chronic lymphatic leukaemia, respectively) complicated with severe thrombocytopenia (platelet count < 10 × $10^9$/l) were given factor VIIa purified from human plasma principally according to the method described by Hedner and Kisiel ( supra).

The first patient was treated in association with a profuse nose bleeding The bleeding time (BT) according to Duke was > 15 minutes before the injection of the factor VIIa as a result of the severe thrombocytopenia. A dose of 100 u/kg b.w. (2 μg/kg b.w.) was given intravenously and 15 minutes after the completion of the injection the Duke BT was normalized (4 minutes, normal range < 5 minutes). The platelet count stayed the same (10 × $10^9$/l) throughout the observation time. The nose bleeding stopped promptly and the clots formed could be removed without any bleeding. A small rebleeding started later but stopped spontaneously. No influence on the pulse, temperature, or blood pressure was observed The factor VII level in plasma rose from 0.66 u/ml to 2.07 u/ml and was again 0.60 at 8 hours after the injection. No influence on the plasma level of factor X (1.12 u/ml before and 1.12 u/ml after the injection) was seen remaining at the same level for the 8 hours observation time. No fibrin/fibrinogen degradation products appeared in the circulation and the ethanol gelation test stayed negative throughout. Furthermore, no changes in ATIII or α $_2$AP were observed.

Patient No. 2 also has a platelet count of < 10 × $10^9$/l and a Duke BT of > 15 minutes before the injection of factor VIIa. The patient bled profusely from the Duke incision in the ear, a bleeding that had to be stopped by manual compression and the local application of thrombin. Factor VIIa in a pure form was given intravenously in a dose of about 100 u/kg b.w. (2 μg/kg b.w.) and the Duke BT was repeated 15 minutes after the completion of the injection. The Duke BT was then 10 minutes and the formation of a visible clot was observed on the site of the incision. No change of the platelet count was recorded and no influence on the pulse, blood pressure, or body temperature occurred. The plasma level of factor VII rose from 0.57 u/ml to 2.17 u/ml. No change in the factor X level (0.73 u/ml before and 0.81 u/ml after the injection) and no change in ATIII or α $_2$AP was seen.

In summary, purified factor VIIa injected intravenously shortened the prolonged BT in patients with severe thrombocytopenia. In parallel, an increase of the plasma level of factor VII was observed. No signs of a general effect on the coagulation mechanism was observed.

From the foregoing it will be appreciated that, although specific embodiments of the invention have been described herein for purposes of illustration, various modifications may be made without deviating from the spirit and scope of the invention.

The features disclosed in the foregoing description and in the following claims may, both separately and in any combination thereof, be material for realising the invention in diverse forms thereof.

## Claims

1. The use of factor VIIa in the preparation of a composition for treating patients suffering from bleeding disorders not caused by clotting factor defects or clotting factor inhibitors, which composition comprises factor VIIa in an effective haemostatic amount.

2. The use according to Claim 1, wherein the composition is for treating patients suffering from bleeding in association with tissue damage.

3. The use according to Claim 1, wherein the composition is for treating patients suffering from thrombocytopenia or platelet disorders.

4. The use according to Claim 1, wherein the composition is for treating patients suffering from gastrointestinal bleeding.

5. The use according to Claim 1, wherein the composition is for treating patients suffering from nasal-oral bleeding.

6. The use according to Claim 1, wherein the composition is for treating patients suffering from bleeding associated with severe tissue damage.

7. The use according to Claim 1, wherein the composition is for treating patients suffering from bleeding associated with surgery.

## Revendications

1. Utilisation du facteur VIIa dans la préparation d'une composition pour traiter des patients atteints de troubles hémorragiques non provoqués par des déficiences du facteur de coagulation ou des inhibiteurs du facteur de coagulation, laquelle composition comprend le facteur VIIa dans une quantité hémostatique efficace.

2. Utilisation selon la revendication 1, dans laquelle la composition sert à traiter des patients présentant des saignements liés à des lésions tissulaires.

3. Utilisation selon la revendication 1, dans laquelle la composition sert à traiter des patients atteints de thrombocytopénie ou de troubles des plaquettes.

4. Utilisation selon la revendication 1, dans laquelle la composition sert à traiter des patients présentant des saignements gastro-intestinaux.

5. Utilisation selon la revendication 1, dans laquelle la composition sert à traiter des patients présentant des saignements du nez et de la bouche.

6. Utilisation selon la revendication 1, dans laquelle la composition sert à traiter des patients présentant des saignements liés à de graves lésions tissulaires.

7. Utilisation selon la revendication 1, dans laquelle la composition sert à traiter des patients présentant des saignements liés à une intervention chirurgicale.

## Patentansprüche

1. Verwendung von Faktor VIIa bei der Herstellung einer Zubereitung zur Behandlung von Patienten, die an Blutungsstörungen leiden, die nicht von Gerinnungsfaktor-Defekten oder Gerinnungsfaktor-Inhibitoren hervorgerufen sind, wobei die Zubereitung Faktor VIIa in einer wirksamen hämostatischen Menge enthält.

2. Verwendung nach Anspruch 1, dadurch gekennzeichnet, daß die Zubereitung für die Behandlung von Patienten ist, die an Blutung in Verbindung mit Gewebeschädigung leiden.

3. Verwendung nach Anspruch 1, dadurch gekennzeichnet, daß die Zubereitung für die Behandlung von Patienten ist, die an Thrombozytopenie oder Thrombozytenstörungen leiden.

4. Verwendung nach Anspruch 1, dadurch gekennzeichnet, daß die Zubereitung für die Behandlung von Patienten ist, die an gastrointestinaler Blutung leiden.

5. Verwendung nach Anspruch 1, dadurch gekennzeichnet, daß die Zubereitung für die Behandlung von Patienten ist, die an nasal-oraler Blutung leiden.

6. Verwendung nach Anspruch 1, dadurch gekennzeichnet, daß die Zubereitung für die Behandlung von Patienten ist, die an mit schwerer Gewebeschädigung verbundener Blutung leiden.

7. Verwendung nach Anspruch 1, dadurch gekennzeichnet, daß die Zubereitung für die Behandlung von Patienten ist, die an mit einem chirurgischen Eingriff verbundener Blutung leiden.